# EUROPEAN PATENT APPLICATION

(11) **EP 1 595 872 A1**
(43) Date of publication of application: **16.11.2005**
(21) Application number: 04011596.6
(22) Date of filing: 14.05.2004
(51) Int. Cl.: C07D 265/28, C07D 279/36, C09B 19/00, C09B 21/00, G01N 33/533

(54) **Fluorescent compounds for monitoring protein-lipid binding**

(71) Applicant: EUROPÄISCHES LABORATORIUM FÜR MOLEKULARBIOLOGIE (EMBL), D-69117 Heidelberg (DE)
(72) Inventor: Schultz, Carsten, 69117 Heidelberg (DE); Wichmann, Oliver, 69126 Heidelberg (DE); Black, Shannon, 69126 Heidelberg (DE)
(74) Representative: Huhn, Michael

(57) **Abstract**

The invention refers to compounds according to formula I 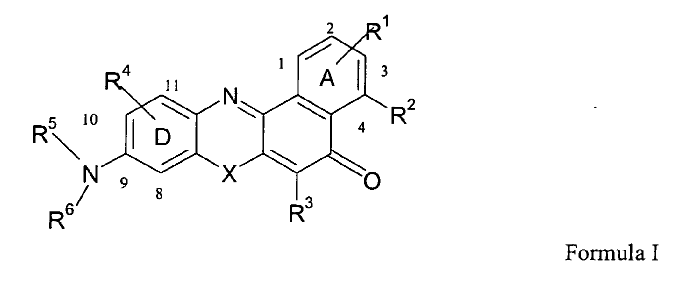 wherein
R¹ is independently in position 2', 3', or both of the A ring of the compound and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, linear or branched -O-(CH₂)ₘ-CH(NH₂)-COOH with m = 1-6, preferably m= 2-4, or any combination thereof,
R² and R³ independently are F, Cl, Br, I, H, CN, -CH=CH-alkyl, -C≡C-alkyl, or an aromatic compound,
X is O or S,
R⁴ is in position 8', 10', or 11' of the D ring of the compound and is H or (CH₂)ₙ-CH₃ with n = 0-6, COOH, CN, or halogen
R⁵ and R⁶ independently are ethyl-, methyl-, H, or -(CH₂)₃ connected to position 8 or 10 of the D ring of the fluorescent dye,
except compounds according to formula I wherein R¹ is 2' or 3' - hydroxyl or 2' -O-(CH₂)₅-COOH, R², R³, and R⁴ are H, X is O, and R⁵ and R⁶ are ethyl.

The invention further refers to the use of the compounds according to formula I as environmentally sensitive fluorescent dyes. They particularly lend themselves to measure the binding of ligands to lipid binding sites of proteins. They can also be used in an assay for performing this measurement.

## Description

The invention refers to compounds according to formula I 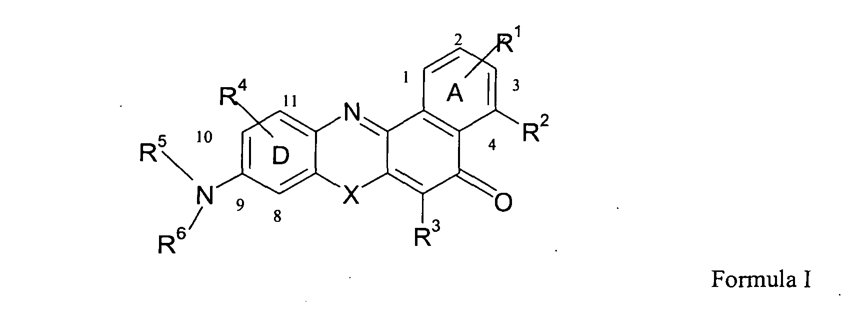 wherein
R¹ is independently in position 2', 3', or in both positions, of the A ring and is hydroxyl, linear or branched -O-alkyl wherein the alkyl group has 1-12, preferably 3-8, more preferably 4 carbon atoms, -O-(alkyl)COOH wherein the alkyl group has 1-12, preferably 3-8, more preferably 4 carbon atoms, -O-(aryl)COOH, -O-aryl, linear or branched -O-(CH₂)ₘ-CH(NH₂)-COOH with m = 1-6, preferably m= 2-4, or any combination thereof;
R² and R³ independently are F, Cl, Br, I, H, CN, -CH=CH-alkyl, -C≡C-alkyl, or aryl,
X is O or S,
R⁴ is in position 8', 10', or 11' of the D ring of the compound and is H or (CH₂)ₙ-CH₃ with n = 0-6, COOH, CN, or halogen
R⁵ and R⁶ independently are ethyl-, methyl-, H, or -(CH₂)₃ connected to position 8 or 10 of the D ring.

The invention further refers to the use of the compounds according to formula I as environmentally sensitive fluorescent dyes. They particularly lend themselves to measure the binding of ligands to lipid binding sites of proteins. They can also be used in an assay for performing this measurement.

Binding of proteins to lipids is a crucial event in translocation of proteins within the cell, e.g. from the cytosol to the mitochondrial matrix, as well as lipid and drug transport. The proteins usually have hydrophobic cavities referred to as lipid binding sites. From the amino acid sequence the specificity of the binding site for a particular lipid cannot be deduced. Especially in the post-genomic era it is important to determine protein-lipid interaction in order to find physiological functions of proteins. Due to the heterogeneous nature of biological membranes, the specificity of lipid-protein interactions is difficult to investigate with native material. Typically, artificial membranes or their substitutes like liposomes or micelles are employed instead, which may lead to unsatisfactory results. It would be desirable to study protein-lipid interaction by adding a single species to the protein.

The study of specific lipid-protein binding is particularly crucial under pharmacological aspects, since many drugs bind to physiological proteins such as albumin, thus replacing endogenous ligands as well as other drugs which can lead to strong, unpredictable side effects and in addition affects the pharmacokinetic properties of the drug.

One approach to investigate specific lipid-protein interaction involves environmentally sensitive fluorescent dyes that change fluorescence properties depending on the hydrophobicity of the surroundings. A known fluorescent dye with these properties is nile red, a dye usually employed to stain lipid droplets in cells. The structure of Nile red forms the basis of formula I with R¹, R², R³, and R⁴ = H, and R⁵ and R⁶ = ethyl.

A main disadvantage of nile red in studying lipid-protein interaction in whole cells is a strong quenching effect of the fluorescence by cytochrome c (Bertsch et al., 2003, Analytical Biochemistry 313:187-195). In addition, measurement of lipid-protein interaction with nile red *in vitro* is hindered by the fact, that nile red is not water-soluble and the fluorescence spectrum is not convenient for automated measurement.

The object of the invention is thus to provide environmentally sensitive fluorescent dyes which alter the emission wave length depending on hydrophobicity of the surrounding and can be used for determination of lipid-protein interaction in an easy, cheap and very fast high throughput screening (HTS) assay *in vitro.*

The object is solved by providing compounds according to formula I which change their fluorescence properties depending on the hydrophobicity of the environment characterised in that these fluorescent dyes show excitation and emission at wavelengths that are longer than e.g. those of e.g. unsubstituted nile red, facilitating their use in automatic applications.

Furthermore, the fluorescent dyes according to the invention containing hydrophilic substituents are water-soluble at the concentrations employed in the competitive binding assay to measure binding of ligands to lipid binding sites of proteins according to the invention. In addition, all fluorescent dyes according to the invention with R⁵ and R⁶ both H can be bound covalently to glass or plastic surfaces of e.g. multi well plates, plate reader plates, micro array chips, or microfluidic devices. These features both contribute to the convenient use of the fluorescent dyes according to formula I in automatic applications.

The object is further solved by providing an assay for identifying lipid binding sites on proteins using the fluorescent dyes according to the invention. The invention further provides a competitive binding assay to measure ligands binding to lipid binding sites of proteins. Both assays can be performed either with fluorescent dyes in aqueous solution or with fluorescent dyes covalently bound to the surfaces mentioned above.

The object is further solved by providing a competitive binding assay according to the invention using the new environmentally sensitive fluorescent dyes which is suitable for an easy, cheap, and very fast high throughput screening for ligands binding to lipid binding sites of proteins.

Increased water-solubility of the fluorescent dyes according to formula I as compared to e.g. nile red, was achieved by coupling them via 2'-O- or 3'-O- on the A-ring of the fluorescent dye with fatty acids, or amino acids of variable lengths (Briggs *et al*. (1997) J. Chem. Soc., Perkin Trans., 1:1051). Butyric acid derivatives are preferred due to sufficient water solubility. A compound according to formula I exemplary for increased water solubility is nile red butyric acid (NRBA), wherein in formula I R¹ is in position 2' of the A ring of the fluorescent dye and is -O-(CH₂)₃-COOH, R², R³, and R⁴ each are H, X is O, and R⁵ and R⁶ each are ethyl.

Surprisingly, insertion of halogen atoms at any position on the fluorescent dye led to a shift of the excitation/emission of that dye to longer wave lengths which are more suitable for automated application. Analogously, substitution of the benzo[a]phenoxazin by benzo[a]phenothiazin and further insertion of a methyl group or any other electron donor group on the D-ring of the fluorescent dye led to a comparable shift of the excitation/emission of that dye to longer wave lengths.

One preferred embodiment of the invention exemplifying halogenation includes thus compounds according to formula I wherein R¹ is independently in position 2', 3', or both of the A ring of the fluorescent dye and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, linear or branched -O-(CH₂)ₘ-CH(NH₂)-COOH with m = 1-6, preferably m= 2-4, or any combination thereof, R² and R³ are independently F, Cl, Br, I or H but not both H, X is O, R⁴ is H, and R⁵ and R⁶ independently are ethyl-, methyl-, H, or -(CH₂)₃ connected to position 8 or 10 of the D ring of the fluorescent dye.

Examples for fluorescent dyes containing halogen atoms are neckar yellow butyric acid (NYBA), wherein in formula I R¹ is in position 2' of the A ring of the fluorescent dye and is -O-(CH₂)₃-CH₃, R² and R⁴ each are H, R³ is F, X is O, and R⁵ and R⁶ each are ethyl; fluorescent dyes according to formula I wherein R¹ is in position 3' of the A ring of the fluorescent dye and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, linear or branched -O-(CH₂)ₘ-CH(NH₂)-COOH with m = 1-6, preferably m= 2-4, R² and R⁴ each are H, R³ is Br, X is O, and R⁵ and R⁶ each are ethyl (e.g. compound 4 and 9 according to table 1); and fluorescent dyes according to formula I wherein R¹ is in position 3' of the A ring of the fluorescent dye and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, linear or branched -O-(CH₂)ₘ-CH(NH₂)-COOH with m = 1-6, preferably m= 2-4, R² and R³ each are Br, R⁴ is H, X is O, and R⁵ and R⁶ each are ethyl (e.g. compound 6 according to table 1).

In one embodiment of the present invention, compounds according to formula I wherein R¹ is 2' or 3' - hydroxyl or 2' -O-(CH₂)₅-COOH, R², R³, and R⁴ are H, X is O, and R⁵ and R⁶ are ethyl, are excluded.

Another preferred embodiment of the invention exemplifying the substitution of the benzo[a]phenoxazin by benzo[a]phenothiazin and the further insertion of any electron donor group on the D-ring of the fluorescent dye includes compounds according to formula I wherein R¹ is in position 2', 3', or both of the A ring of the fluorescent dye and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, linear or branched -O-(CH₂)ₘ-CH(NH₂)-COOH with m = 1-6, preferably m= 2-4, or any combination thereof, X is S, R² and R³ independently are F, Cl, Br, I, H, CN, -CH=CH-alkyl, -C≡C-alkyl, or an aromatic compound, R⁴ is H or (CH₂)ₙ-CH₃ with n = 0-6, COOH, CN, or halogen, and R⁵ and R⁶ independently are ethyl-, methyl, H, or -(CH₂)₃ connected to position 8 or 10 of the D ring of the fluorescent dye. Examples for this preferred embodiment are fluorescent dyes according to formula I, wherein R¹ is in position 3' of the A ring of the fluorescent dye and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, linear or branched -O-(CH₂)ₘ-CH(NH₂)-COOH with m = 1-6, preferably m= 2-4, R² and R³ each are H, R⁴ is methyl, X is S, and R⁵ and R⁶ each are ethyl (e.g. compounds 7, 10, and 8 according to table 1).

A further preferred embodiment comprises environmentally sensitive fluorescent dyes according to formula I wherein R¹ is independently in position 2', 3', or both of the A ring of the fluorescent dye and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, linear or branched -O-(CH₂)ₘ-CH(NH₂)-COOH with m = 1-6, preferably m= 2-4, or any combination thereof, R² and R³ independently are F, Cl, Br, I, H, CN, -CH=CH-alkyl, -C≡C-alkyl, or an aromatic compound, X is O or S, R⁴ is H or (CH₂)ₙ-CH₃ with n = 0-6, COOH, CN, or halogen, and R⁵ and R⁶ each are H. These fluorescent dyes can be covalently bound via a spacer coupled to the amino group of the D-ring of the fluorescent dye to glass or plastic surfaces of for example multi well plates, plate reader plates, micro array chips, or microfluidic devices, which can then be employed for high throughput screening for ligands binding to lipid binding sites of proteins.

The environmentally sensitive fluorescent dyes according to the present invention are synthesized using standard methods as described exemplary in examples 1 to 4. Compounds with a 6-Bromo or 6-Chloro substitution could be used to make compounds with a 6-Fluoro or 6-Iodo substitution as well. These halogenated fluorescent dyes (particularly the iodo-, chloro- and bromo-compounds) could be used for the synthesis of further fluorescent dyes via Suzuki, Stille or Sonogashira methodology.

Some exemplary fluorescent dyes according to the invention together with analytical data, as well as values for excitation and emission are included in table 1. Compounds 1 and 5, which are the 2'-hydroxy and 3'-hydroxy derivatives of nile red, respectively, are included as reference compounds. The excitation and emission values of the fluorescent dyes were measured in methanol, which simulates a protein-environment, thus yielding a shift of the emission to shorter wave lengths. For comparison, excitation and emission values for compound 10 were also measured in HEPES buffer (i.e. under initial, aqueous assay conditions). In this aqueous environment, the excitation is 620 nm and the emission is 688 nm compared to an emission at 662 nm in methanol.

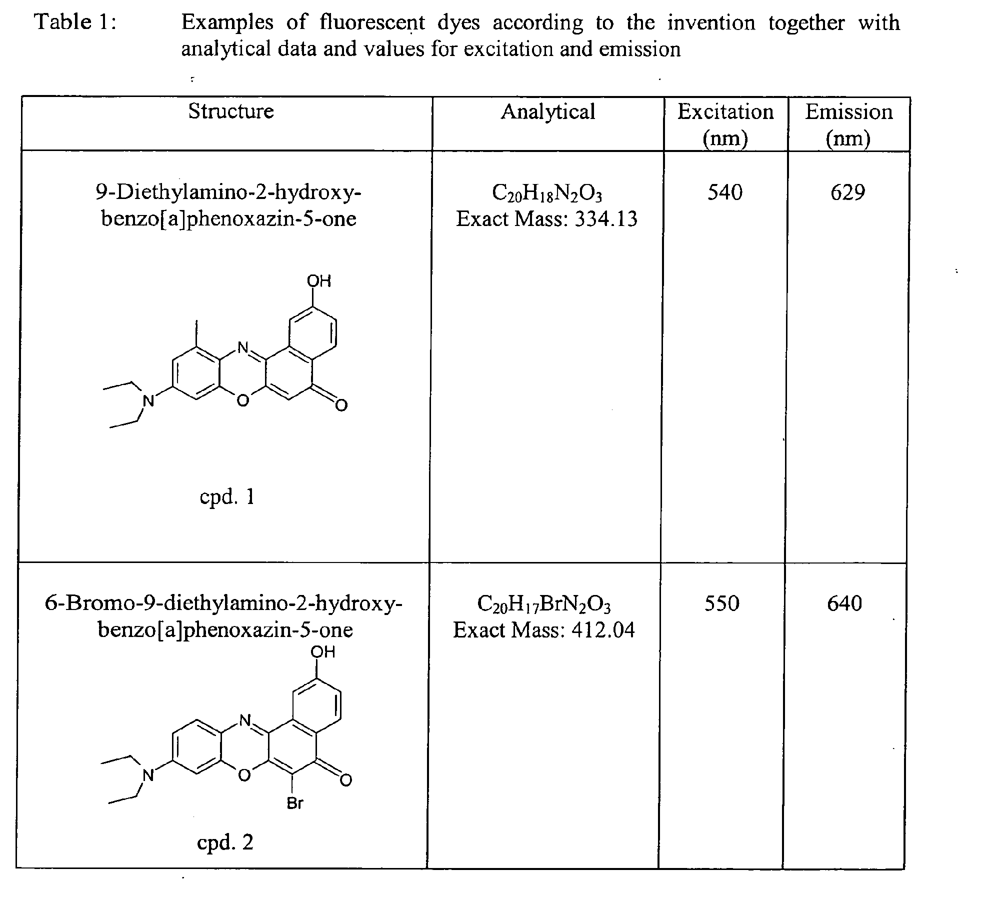

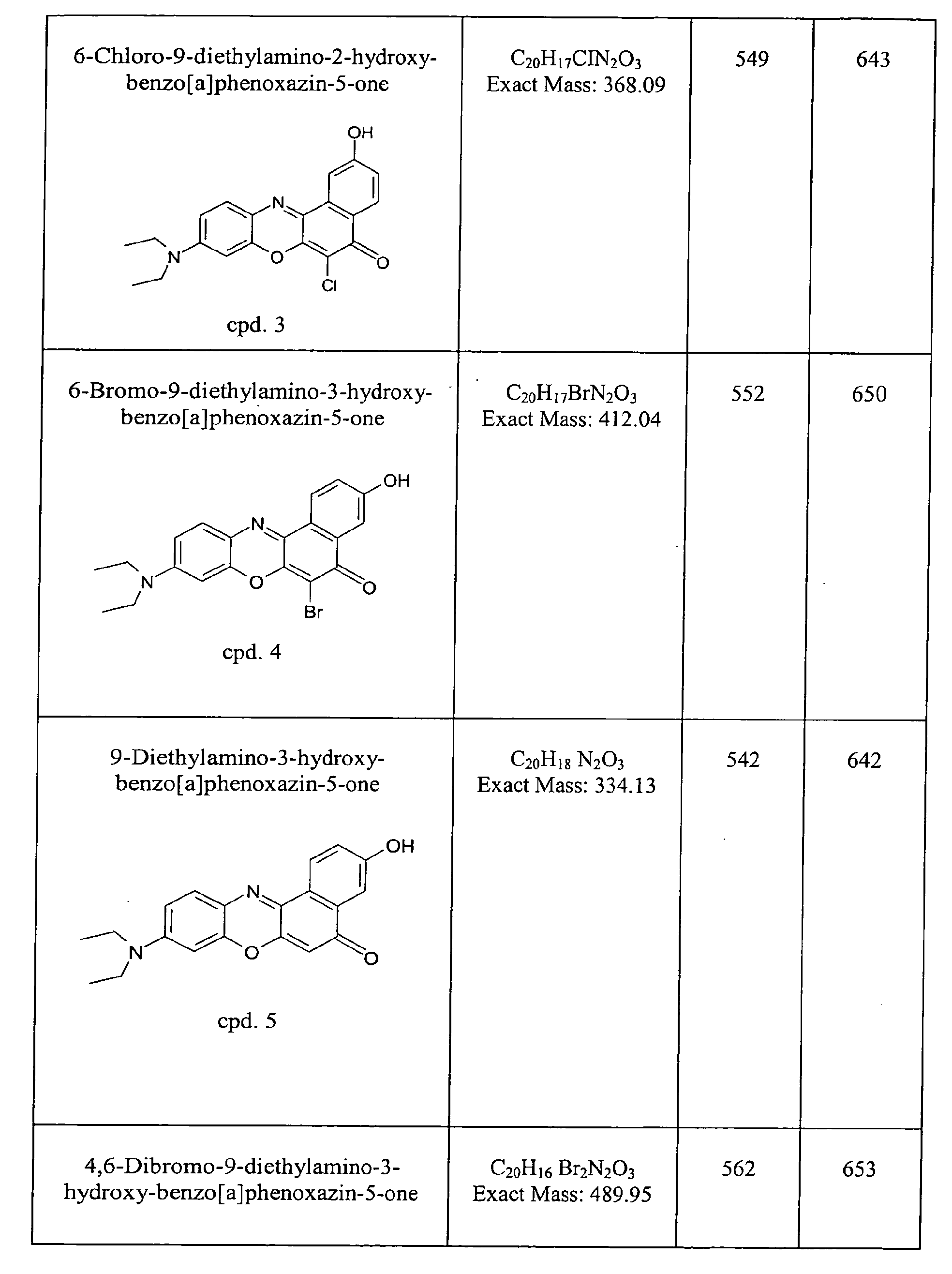

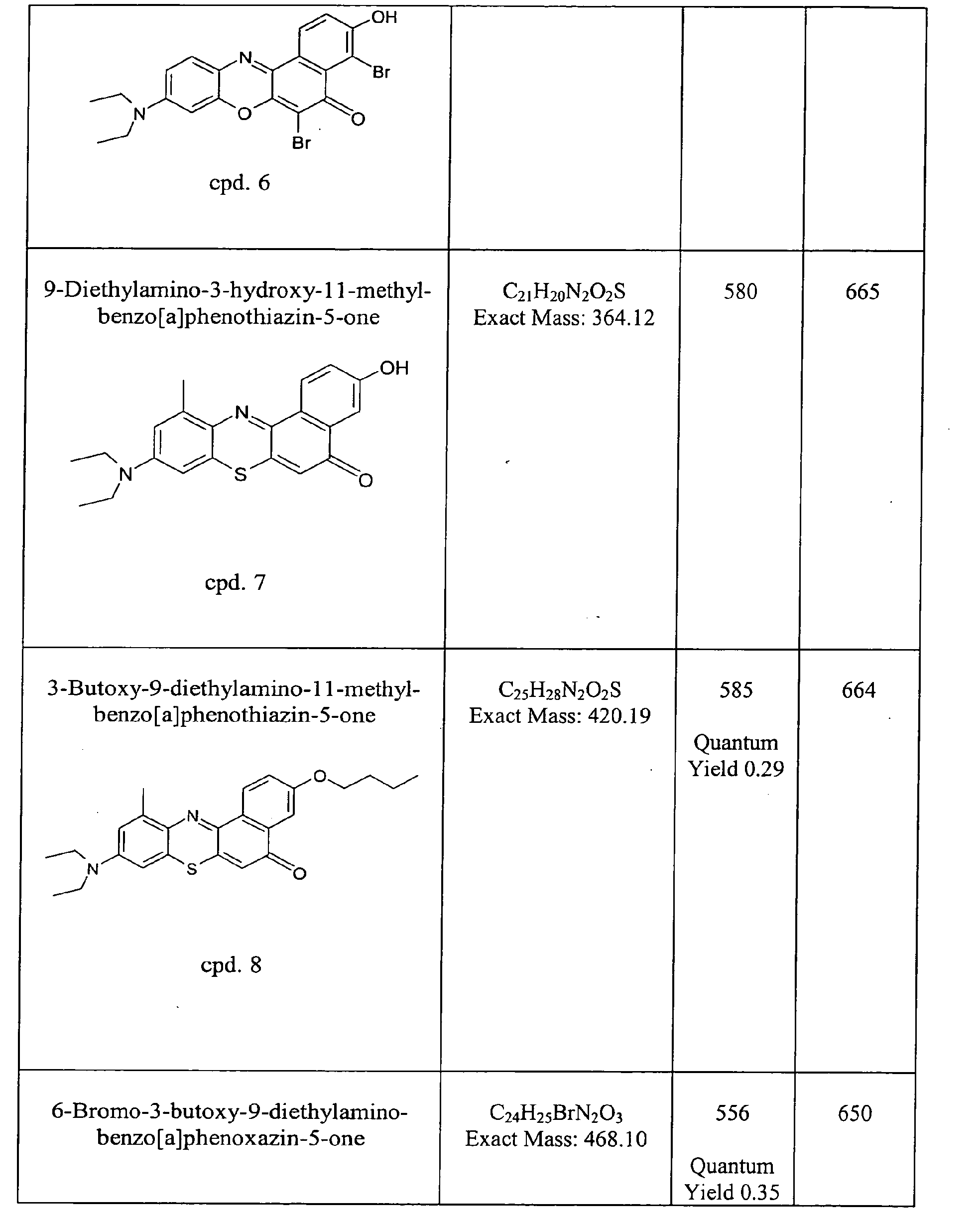

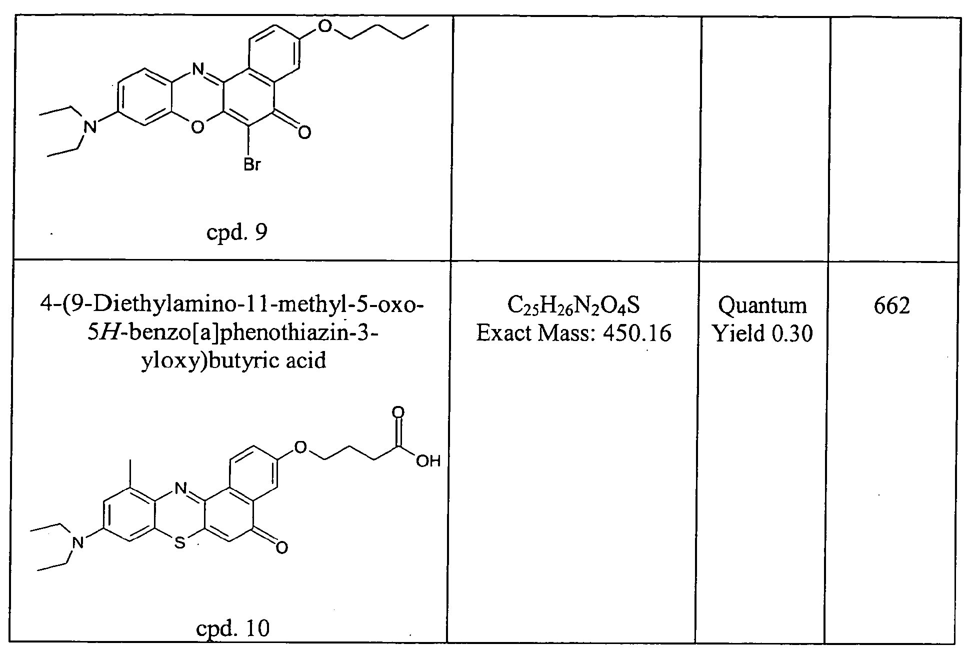

The invention further relates to an assay for identifying lipid binding sites on proteins using the compounds according to formula I comprising
a) dissolving a compound according to formula I in an aqueous solvent,
b adding the protein to be tested for containing a lipid binding site in various concentrations,
c) measuring the intensity of fluorescence emission at the wave length of maximal emission of the fluorescent dye in non-aqueous solution such as methanol.

The fluorescent dye will bind to any lipid-binding site of the protein, and the resulting increase in the hydrophobicity of the surrounding of the dye will result in an increase in the intensity of the fluorescence and a shift in the emission to a shorter wavelength. The emission-wavelength of a compound according to formula I in a hydrophobic surrounding can be determined in a non-aqueous solution such as methanol.

The invention further relates to a competitive binding assay to measure the binding of ligands to lipid binding sites of proteins using the compounds according to formula I comprising
a) dissolving a compound according to formula I in an aqueous solvent,
b) adding a protein containing at least one lipid binding site,
c) adding the ligand to be tested in various concentrations,
d) measuring the intensity of fluorescence emission at the wave length of maximal emission of the fluorescent dye in non-aquous solution such as methanol.

Depending on the strength of the binding of the ligand to the lipid binding site of the protein, the ligand releases the fluorescent dye from the hydrophobic lipid binding site. This can be measured by a decrease of the intensity of fluorescence at the emission wavelength of the dye in hydrophobic surrounding, as imitated by dissolving the dye in methanol.

In a preferred embodiment of the competitive binding assay, a fluorescent dye covalently linked to glass or plastic surfaces of for example multi well plates, plate reader plates, micro array chips, or microfluidic devices is applied in step a) of the assay, in step b) the surface is loaded with a protein containing at least one lipid binding site, step c) is carried out as described, and in step d) the intensity of fluorescence emission at the wave length of maximal emission of the fluorescent dye in non-aquous solution such as methanol is measured in an appropriate reader.

The present invention further relates to the use of the competitive binding assay for high throughput screening for ligands useful as competitive inhibitors of protein-lipid interaction. These competitive inhibitors can be used wherever a specific lipid-protein interaction leads to a pathophysiological state in patients.

The invention further relates to the use of the competitive binding assay to study pharmacokinetics of drug binding to lipid binding sites on proteins. As mentioned above, many drugs bind to physiological proteins such as albumin, thus releasing endogenous ligands as well as other drugs which can lead to strong, unpredictable side effects and in addition affects the pharmacokinetic properties of the drug.

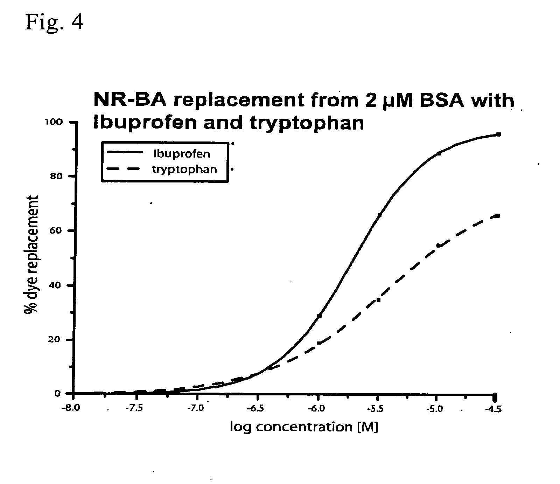

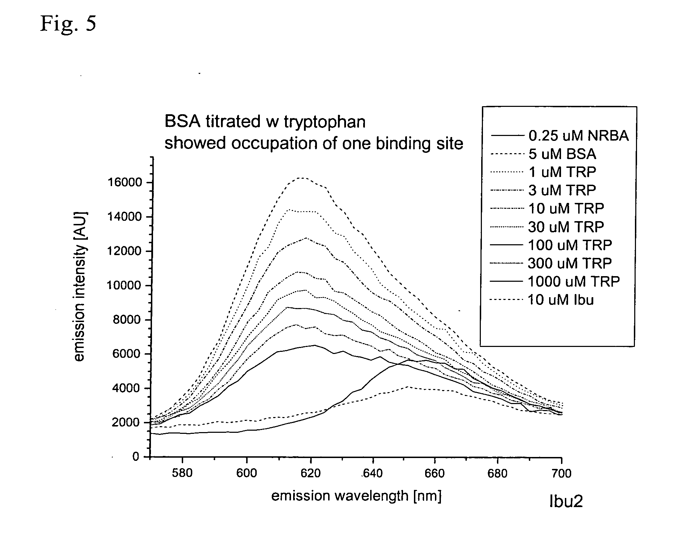

Drugs which strongly bind to the lipid binding side of proteins will thus release drugs which show only a weak binding to endogenous proteins, raising the blood levels of the latter. The number of patients, especially the elderly, taking several drugs at a time, often prescribed by different doctors, is increasing. For this reason there is a strong need to study possible interactions of drugs. By comparing the respective binding properties of different drugs using the fluorescent dyes and the assay according to the invention (Fig. 4 and 5), it is possible to predict drug interaction due to competitive binding to the lipid binding sites of endogenous proteins and thus prevent side effects which are prone to occur especially in patients taking several drugs at a time.

"Environmentally sensitive fluorescent dyes" within the meaning of the invention are fluorescent dyes that change fluorescence properties depending on the hydrophobicity of the surroundings.

"Multi well plates" within the meaning of the invention are plates of different material, mostly plastic or glass, comprising at least 6 wells.

"Plate reader plates" within the meaning of the invention are plates of different material, mostly plastic or glass, comprising at least 96 wells, but also several hundred or up to several thousand wells. An example for plate reader plates are micro titre plates.

"Micro array chips" within the meaning of the invention are solid phase carriers of different materials in the form of small-sized chips, e.g. in the size of cover slips.

"Microfluidic devices" within the meaning of the invention are miniaturized modules for unit operations in chemical technology which can be applied in high throughput screening assays.

"High throughput screening" within the meaning of the invention comprises screening assays allowing the screening of at least 96 different compounds or different concentrations at a time.

### Figures

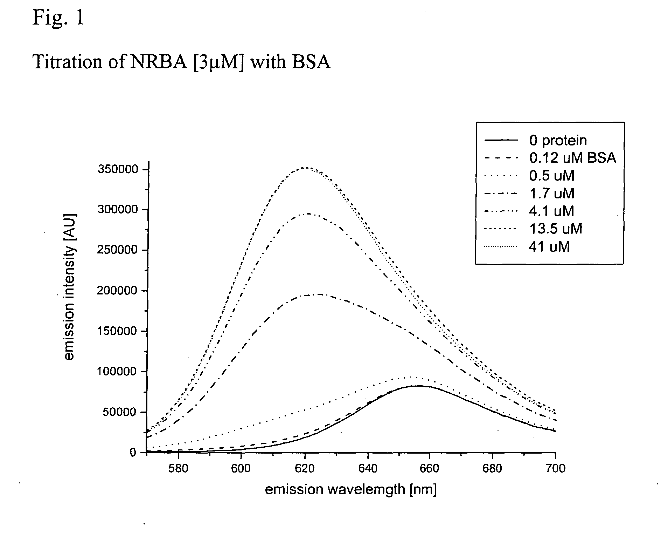

Fig. 1 shows the titration of nile red butyric acid (NRBA) [3 µM] with bovine serum albumin (BSA) which leads to a shift in the emission to a shorter wavelength and an increase in the intensity of the fluorescence.

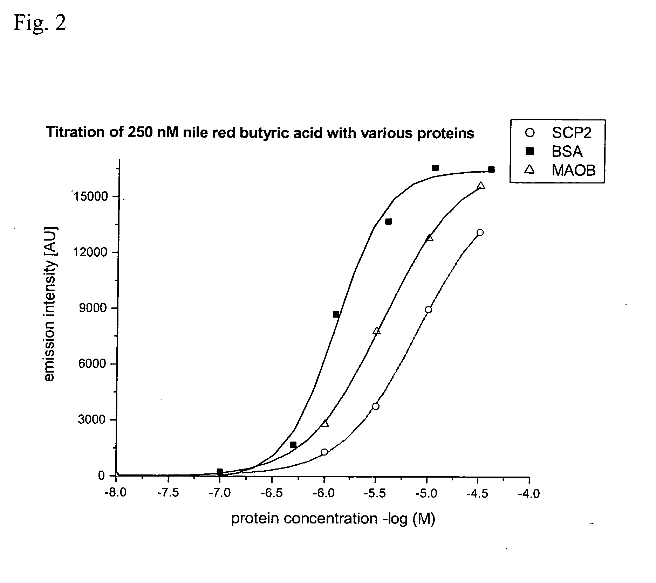

Fig. 2 shows the titration of NRBA [250 nM] with different proteins: BSA, the peroxisomal matrix protein (SCP2), and monoamine oxidase B (MAO B). BSA had the strongest binding to NRBA, SCP2 the weakest.

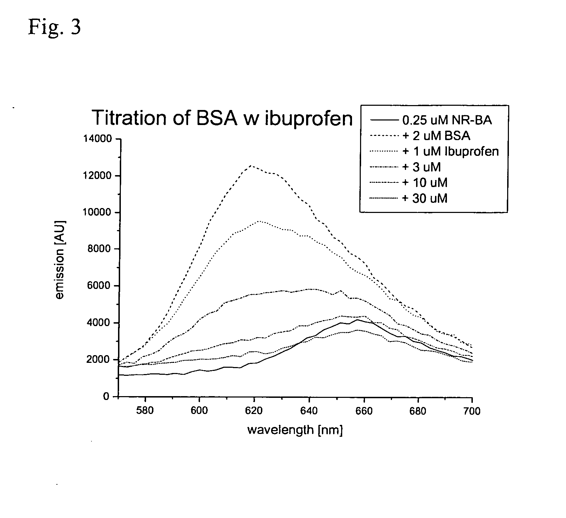

Fig. 3 shows the replacement of NRBA [250 nM] from 2 µM BSA with ibuprofen.
Fig. 4 shows a comparison of the replacement of NRBA from 2 µM BSA with ibuprofen and tryptophan (the endogenous ligand of albumin). Ibuprofen was able to bind to both lipid binding sites of albumin while tryptophan only replaced one molecule of the dye.
Fig. 5 shows the experiment of Fig. 4 in a diagram of emission intensity vs. emission wavelength. In contrast to ibuprofen [10 µM], even 1 mM of tryptophan was not able to release all the dye from BSA.

### Examples

### Example 1

### Synthesis of 6-Bromo-9-diethylamino-3-hydroxy-benzo[a]phenoxazin-5-one (compound 4 in table 1)

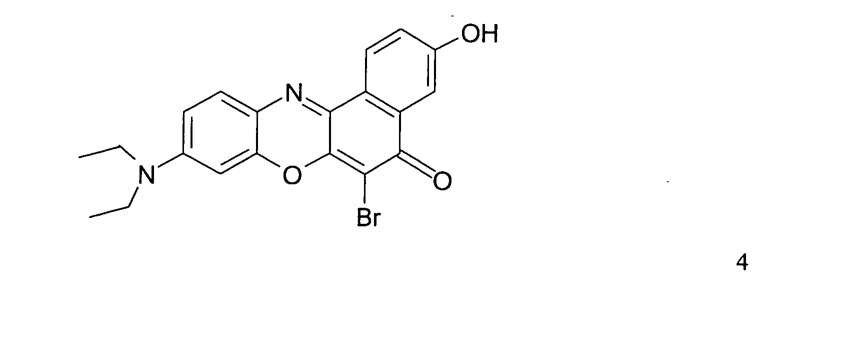

Bromine (0.10 ml, 1.9 mmol) was added to a solution of 9-Diethylamino-3-hydroxybenzo[a]phenoxazin-5-one (0.20 g, 0.6 mmol) in 100 ml chloroform and the solution allowed to stir at RT, under nitrogen for 2h. The solution was then concentrated under reduced pressure (30 °C) and the residue further purified by column chromatography [methanol/dichloromethane (3:97) - methanol/dichloromethane (10:90)] to give 6-Bromo-9-diethylamino-3-hydroxy-benzo[a]phenoxazin-5-one (0.06 g, 25%), (FAB⁺) 413 (60%).

### Example 2

### Synthesis of 4,6-Dibromo-9-diethylamino-3-hydroxy-benzo[a]phenoxazin-5-one (Compound 6)

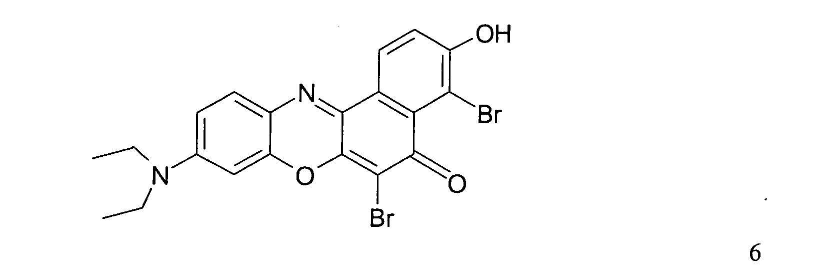

Bromine (0.20 ml, 3.6 mmol) was added to a solution of 9-Diethylamino-3-hydroxybenzo[a]phenoxazin-5-one (0.20 g, 0.6 mmol) in 100 ml chloroform and the solution allowed to stir at 40 °C, under nitrogen for 2h. The solution was then concentrated under reduced pressure (40 °C) and the residue further purified by column chromatography [methanol/dichloromethane (1:99) ― methanol/dichloromethane (5:95)] to give 4,6-Dibromo-9-diethylamino-3-hydroxy-benzo[a]phenoxazin-5-one (0.085 g, 29%), (FAB⁺) 493 (100%).

### Example 3

### Synthesis of 9-Diethylamino-3-hydroxy-11-methyl-benzo[a]phenothiazin-5-one (compound 7)

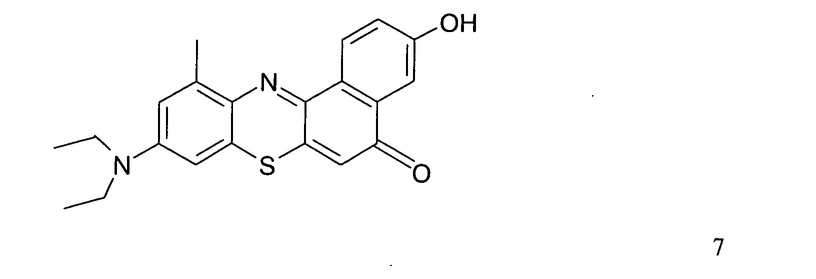

Thiosulfuric acid S-(2-amino-5-diethylamino-3-methyl-phenyl) ester (0.50 g, 1.7 mmol) (*Photochem. and Photobiol*., 1998, 67, 343) and 1,7 dihydroxynaphthalene (0.28 g, 1.7 mmol) were dissolved in dimethylsulfoxide (10 ml) and stirred at RT for 45 min. Potassium dichromate (0.51 g, 1.73 mmol) was added and the reaction stirred for a further 20 min. A mixture of methanol (200 ml) and 1N HCl (20 ml) was then added and the solution stirred for a further 1 h at RT. The excess methanol was then removed under reduced pressure and aq. sodium bicarbonate was added to the residue in dimethylsulfoxide. The mixture was sealed and left at RT for 18 h. The resultant precipitate was removed by filtration, washed with water and dried under high vacuum to yield 9-Diethylamino-3-hydroxy-11-methyl-benzo[a]phenothiazin-5-one (0.46 g, 73 %), (FAB⁺) 365 (20%).

### Example 4

The attachment of any acid to the 2' or 3' Hydroxygroup on the A ring of the fluorescent dyes was accomplished using the methodology as described in *Briggs et al*. (1997) J. Chem. Soc., Perkin Trans., 1:1051.

### Example 5

### Protocol for nile red lipid binding assay

0.25 µl nile red dye stock solution (10 µM) was added to 90 µl HEPES buffer (final concentration 30 nM) and the mixture was transferred to a fluorimeter cuvette. For determining the submaximal concentration of dye binding to the protein, the latter was titrated until no further fluorescence increase at 620 nm could be detected.

For a typical lipid binding assay a protein concentration that showed 90% of the maximal fluorescence increase was selected. Increasing concentrations of lipid or drug were added to replace the dye in a dose-dependent fashion. Fluorescence values at 620 nm were corrected for basal fluorescence at 620 nm prior to protein addition as well as for dilution effects from adding increasing ligand concentrations. The starting fluorescence value minus the value at a given ligand concentration was plotted as % replacement.

## Claims

1. A compound according to formula I 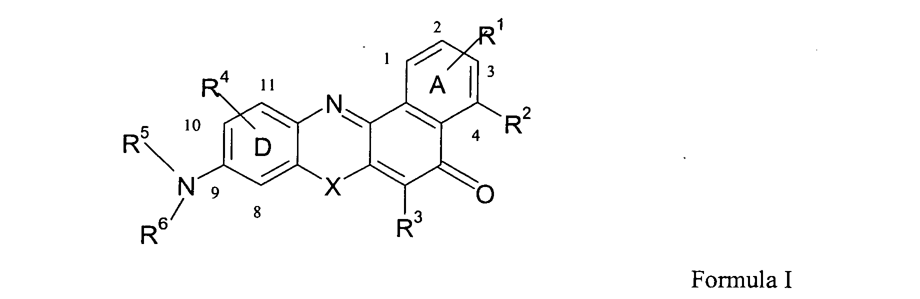 wherein
R¹ is independently in position 2', 3', or in both positions, of the A ring and is hydroxyl, linear or branched -O-alkyl wherein the alkyl group has 1-12, preferably 3-8, more preferably 4 carbon atoms, -O-(alkyl)COOH wherein the alkyl group has 1-12, preferably 3-8, more preferably 4 carbon atoms, -O-(aryl)COOH, -O-aryl, linear or branched -O-(CH₂)ₘ-CH(NH₂)-COOH with m = 1-6, preferably m= 2-4, or any combination thereof;
R² and R³ independently are F, Cl, Br, I, H, CN, -CH=CH-alkyl, -C≡C-alkyl, or aryl,
X is O or S,
R⁴ is in position 8', 10', or 11' of the D ring and is H or (CH₂)ₙ-CH₃ with n = 0-6, COOH, CN, or halogen
R⁵ and R⁶ independently are ethyl-, methyl-, H, or -(CH₂)₃ connected to position 8 or 10 of the D ring.

2. A compound according to claim 1 wherein R¹ is independently in position 2', 3', or both of the A ring and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, linear or branched -O-(CH₂)ₘ-CH(NH₂)COOH with m = 1-6, preferably m= 2-4, or any combination thereof, R² and R³ independently are F, Cl, Br, I, H, CN, -CH=CH-alkyl, -C * C-alkyl, or an aromatic compound but not both H, X is O, R⁴ is H, and R⁵ and R⁶ are as defined in claim 1.

3. A compound according to claim 1 wherein
R¹ is independently in position 2', 3', or both of the A ring and is hydroxyl, linear or branched -O-alkyl with 1-12, preferably 3-8, more preferably 4 carbon atoms, - O-carboxylic acid with 1-12, preferably 3-8, more preferably 4 carbon atoms, -O-aromatic compound, linear or branched -O-(CH₂)ₘ-CH(NH₂)-COOH with m = 1-6, preferably m= 2-4, or any combination thereof, X is S, R⁴ is methyl, and R², R³, R⁵, and R⁶ are as defined in claim 1.

4. A compound according to claim 1 wherein R¹ is in position 2' of the A ring and is - O-(CH₂)₃-CH₃, R², R³, and R⁴ each are H, X is O, and R⁵ and R⁶ each are ethyl.

5. A compound according to claim 1 wherein R¹ is in position 2' of the A ring and is - O-(CH₂)₃-CH₃, R² and R⁴ each are H, R³ is F, X is O, and R⁵ and R⁶ each are ethyl.

6. A compound according to claim 1 wherein R¹ is in position 3' of the A ring and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, or linear or branched -O-(CH₂)ₘ-CH(NH₂)-COOH with m = 1-6, preferably m= 2-4, R² and R⁴ each are H, R³ is Br, X is O, and R⁵ and R⁶ each are ethyl.

7. A compound according to claim 1 wherein R¹ is in position 3' of the A ring and is hydroxyl, linear or branched -O-alkyl with 1-12, preferably 3-8, more preferably 4 carbon atoms, -O-carboxylic acid with 1-12, preferably 3-8, more preferably 4 carbon atoms, -O-aromatic compound, or linear or branched -O-(CH₂)ₘ-CH(NH₂)-COOH with m = 1-6, preferably m= 2-4, R² and R³ each are Br, R⁴ is H, X is O, and R⁵ and R⁶ each are ethyl.

8. A compound according to claim 1 wherein R¹ is in position 3' of the A ring and is hydroxyl, linear or branched -O-alkyl with 1-12, preferably 3-8, more preferably 4 carbon atoms, -0-carboxylic acid with 1-12, preferably 3-8, more preferably 4 carbon atoms, -O-aromatic compound, or linear or branched -O-(CH₂)ₘ-CH(NH₂)-COOH with m = 1-6, preferably m= 2-4, R² and R³ each are H, R⁴ is methyl, X is S, and R⁵ and R⁶ each are ethyl.

9. A compound according to one of claims 1 to 8 wherein R¹, R², R³, R⁴, and X are as defined in claim 1 and R⁵ and R⁶ each are H.

10. Compound according to claim 9, covalently bound via a spacer coupled to the amino group of the D-ring to glass or plastic surfaces of for example multi well plates, plate reader plates, micro array chips, or microfluidic devices.

11. Use of a compound according to formula I as environmentally sensitive fluorescent dye.

12. Use of the fluorescent dyes according to one of claims 1 to 10 to identify lipid binding sites on proteins.

13. Use of the fluorescent dyes according to one of claims 1 to 10 to measure the binding of ligands to lipid binding sites of proteins.

14. Assay for identifying lipid binding sites on proteins using the fluorescent dyes according to one of the claims 1-9 comprising
dissolving a compound according to formula I as defined in one of claims 1 to 9 in an aqueous solvent
adding the protein to be tested for containing a lipid binding site in various concentrations
measuring the intensity of fluorescence emission at the wave length of maximal emission of the fluorescent dye in non-aquous solution such as methanol.

15. Competitive binding assay to measure the binding of ligands to lipid binding sites of proteins using the fluorescent dyes according to one of the claims 1-9 comprising dissolving a compound according to formula I as defined in one of claims 1 to 9 in an aqueous solvent,
adding a protein containing at least one lipid binding site,
adding the ligand to be tested in various concentrations,
measuring the intensity of fluorescence emission at the wave length of maximal emission of the fluorescent dye in non-aquous solution such as methanol.

16. The assay according to claim 14 or 15, wherein a compound according to claim 1 is covalently linked to glass or plastic surfaces of for example multi well plates, plate reader plates, micro array chips, or microfluidic devices according to claim 10 is applied, and the intensity of fluorescence emission at the wave length of maximal emission of the fluorescent dye in non-aquous solution such as methanol is measured in an appropriate reader.

17. Use of the competitive binding assay according to claim 15 or 16 to study pharmacokinetics of drug binding to the lipid binding sites of proteins.

18. Use of the competitive binding assay according to claim 15 for high throughput screening for ligands useful as competitive inhibitors of protein-lipid interaction.

19. Competitive inhibitors of protein lipid interactions identified by carrying out the assay according to claim 15 or 16.

20. Kit for identifying competitive inhibitors for protein-lipid interaction comprising a compound according to one of the claims 1 to 10,
any protein with at least one lipid binding site
at least one solvent to solubilize the fluorescent dye and the protein
assay buffer such as HEPES.
